(19) Europäisches Patentamt

European Patent Office

Office européen des brevets

(11) **EP 0 939 317 A2**

(12) **EUROPÄISCHE PATENTANMELDUNG**

(43) Veröffentlichungstag:
**01.09.1999 Patentblatt 1999/35**

(51) Int. Cl.$^6$: **G01N 33/22**, G01N 33/00

(21) Anmeldenummer: 99102102.3

(22) Anmeldetag: **03.02.1999**

(84) Benannte Vertragsstaaten:
**AT BE CH CY DE DK ES FI FR GB GR IE IT LI LU
MC NL PT SE**
Benannte Erstreckungsstaaten:
**AL LT LV MK RO SI**

(30) Priorität: **28.02.1998 DE 19808533**

(71) Anmelder:
- **RUHRGAS AKTIENGESELLSCHAFT
45138 Essen (DE)**
- **N.V. NEDERLANDSE GASUNIE
9700 MA Groningen (NL)**

(72) Erfinder:
- **Jaeschke, Manfred Dr. Dipl.-Phys.
46286 Dorsten (DE)**
- **Schley, Peter Dipl.-Ing.
45131 Essen (DE)**
- **Janssen-van Rosmalen, Renee Dr.ir.
9301 WB Roden (NL)**
- **Schouten, Jan A. Prof. Dr.
1141 GT Monnickendam (NL)**

(74) Vertreter: **Harlacher, Mechthild
Ruhrgas AG,
Abteilung TATP
45138 Essen (DE)**

(54) **Verfahren zur Bestimmung der Gaszusammensetzung von Brenngas**

(57) Der Brennwert und die Normdichte des Brenngases werden erfaßt. Außerdem wird der Kohlenstoffdioxidanteil des Brenngases gemessen. Aus diesen drei Parametern kann die Gaszusammensetzung zuverlässig und ohne Verwendung eines Gaschromatographen bestimmt werden.

EP 0 939 317 A2

**Beschreibung**

Die Erfindung betrifft ein Verfahren zur Bestimmung der Gaszusammensetzung von Brenngas, insbesondere von Erdgas.

Es ist bekannt, daß Gaszusammensetzungen mit einem Gaschromatographen gemessen werden können. Gaschromatographen liefern genaue Meßwerte für die Gaszusammensetzungen, sind jedoch äußerst kostspielig in der Anschaffung und in der Wartung.

Aufgabe der Erfindung ist es, eine kostengünstigere Bestimmung der Gaszusammensetzung von Brenngas zu ermöglichen.

Gelöst wird diese Aufgabe erfindungsgemäß dadurch, daß bei dem eingangs genannten Verfahren

a) der Brennwert oder die Dielektrizitätskonstante des Brenngases unter Referenz- oder Betriebsbedingungen und die Dichte oder die Schallgeschwindigkeit des Brenngases unter Referenz- oder Betriebsbedingungen erfaßt werden und

b) aus den erfaßten zwei Meßsignalen die Gaszusammensetzung abgeleitet wird.

Der Brennwert kann z.B. mit Hilfe von kalorimetrischen Verfahren bestimmt werden, in denen eine kontrollierte Teilstromverbrennung des übertragenen Gasstroms durchgeführt wird. Alternativ kann der Brennwert mit Hilfe von bekannten verbrennungslosen korrelativen Verfahren bestimmt werden. Für die Messung der Dielektrizitätskonstanten, der Dichte sowie der Schallgeschwindigkeit existieren in der Praxis verschiedene bewährte Verfahren, die jeweils ohne besonderen technischen Aufwand zuverlässig und genau durchgeführt werden können. Folglich liefert die Verknüpfung der Meßwerte entsprechende Ergebnisse für die Gaszusammensetzung.

Die auf diese Weise bestimmte Gaszusammensetzung stimmt mit den mit Hilfe der Gaschromatographie bestimmten Anteilen von Erdgas für die Hauptkomponenten Methan, Ethan und Stickstoff besser als 0,1 % überein.

An Stellen, wo die erforderlichen Meßsignale für Abrechnungszwecke bereits erfaßt werden, ist eine zusätzliche Messung der Gaszusammensetzung nicht mehr erforderlich. Allenfalls zur Kontrolle kann in vorgegebenen zeitlichen Abständen, z.B. jährlich, mit einem Gaschromatographen die Gaszusammensetzung gemessen werden. Besonders genaue Ergebnisse lassen sich für Brenngase erzielen, deren Brennwert bei Normbedingungen zwischen 20 und 48 MJ/m$^3$ liegt, deren auf trockene Luft bezogene relative Dichte zwischen 0,55 und 0,9 beträgt, deren Kohlenstoffdioxidanteil kleiner gleich 0,3 ist und deren Wasserstoff- und Kohlenmonoxidanteil kleiner als 0,1 bzw. 0,03 ist. Als Meßbedingungen sind Temperaturen im Bereich von 225 bis 350 K und Drücke kleiner gleich 60 MPa besonders geeignet.

Die Meßgenauigkeit der Gaszusammensetzung kann dadurch weiter erhöht werden, daß im Schritt a) zusätzlich wenigstens eine der Meßgrößen Druck, Temperatur, Kohlenstoffdioxidanteil, Kohlenstoffmonoxid- und Wasserstoffanteil des Brenngases erfaßt wird. Selbstverständlich kann die höchste Meßgenauigkeit bei der zusätzlichen Erfassung sämtlicher zusätzlicher Meßgrößen erzielt werden.

Zum Auffinden einer geeigneten Korrelation zwischen den erfaßten Meßsignalen und der Gaszusammensetzung ist es vorteilhaft, den jeweiligen Verfahrensschritten a) und b) wenigstens einmal mehrere Meßzyklen vorzuschalten, bei denen der Schritt a) mit mehreren Referenzgasen bekannter Zusammensetzung durchgeführt wird. An dem Referenzgas werden dann die für die verschiedenen Varianten der Verfahren benötigten Meßgrößen erfaßt. In diesen Referenzzyklen wird aus dem Verhältnis der erfaßten Meßsignale eine der Zahl der Meßzyklen entsprechende zahl von Referenzsignalmustern in Zuordnung zu den bekannten Zusammensetzungen gespeichert. Das Signalmuster aus einem späteren Meßzyklus an Brenngas unbekannter Zusammensetzung wird mit diesen Referenzsignalmustern zur Zuordnung einer bestimmten Zusammensetzung verglichen.

Zur Erhöhung der Referenzgenauigkeit sollte eine Vielzahl von Referenzzyklen durchgeführt werden, in denen die Anteile der einzelnen Komponenten, möglichst nacheinander, über die jeweils zu erwartenden Anteile der Komponenten am Brenngas variiert werden. Eine eindeutige und genaue Zuordnung einer bestimmten Gaszusammensetzung zu einem in einem Meßzyklus erfaßten Signalmuster eines Brenngases wird durch Interpolation der verschiedenen Referenzsignalmuster erzielt. Die Zusammensetzung der Referenzgase sollte hierbei möglichst getreu den nachfolgend zu messenden Gaszusammensetzungen gewählt werden.

Eine bevorzugte Weiterbildung der Erfindung ist dadurch gekennzeichnet, daß der jeweilige Anteil einer vorgegebenen Anzahl von Alkanen, einschließlich Methan, dadurch bestimmt wird, daß der Anteil der einzelnen Alkane, ausgenommen Methan, mit Hilfe jeweils einer zugehörigen, von einer ausgewählten physikalischen Meßgröße, vorzugsweise dem molaren Brennwert, der Summe der vorgegebenen Alkane abhängigen Funktion bestimmt wird und daß der Methananteil aus der Differenz zwischen dem Anteil der Summe der vorgegebenen Alkane und der Summe der durch die Funktionen bestimmten Anteile der Alkane bestimmt wird.

Als vorgegebene Alkane sollten möglichst alle im Brenngas tatsächlich vorhandenen Alkane gewählt und vorgegeben werden.

Es hat sich gezeigt, daß die Anteile der Alkane bei natürlichen Brenngasen stets in einem bestimmten Verhältnis zueinander stehen, welches lediglich von einer physikalischen Meßgröße, z. B. dem molaren Brennwert, der Summe der vorgegebenen Alkane abhängt. Dies ist offenbar darauf zurückzuführen, daß Erdgase in ihrer natürlichen Form stets eine Gleichgewichtsphase durchlaufen haben, in welcher ihre Gas- oder Flüssigkeitsphasen im Gleichgewicht zueinander standen. Jedoch ist das Verfahren nicht auf die natürlichen Brenngase, und zwar mit oder ohne Kokereigaszusatz, beschränkt. Für synthetische Gase mit Zusätzen oder für Gasgemische mit vielen Komponenten ist die Unsicherheit bei der Bestimmung der Gaszusammensetzung lediglich etwas größer.

Der molare Brennwert der Summe der vorgegebenen Alkane kann beispielsweise wiederum mit Hilfe von Referenzsignalzyklen bestimmt werden. Da bei den Referenzgasen die Zusammensetzung bekannt ist, ist auch ihr molarer Brennwert der Summe der vorgegebenen Alkane bekannt. Folglich kann aus dem Verhältnis der bei den Referenzgasen erfaßten Meßsignale eine der Zahl der Referenzmeßzyklen entsprechende Zahl von Referenzsignalmustern in Zuordnung zu den bekannten molaren Brennwerten der Summe der vorgegebenen Alkane gespeichert werden. Bei einem späteren Meßzyklus kann der molare Brennwert der Summe der vorgegebenen Alkane des Brenngases lediglich durch Vergleich der erfaßten Meßsignale mit den gespeicherten Referenzsignalmustern bestimmt werden.

Die ausgewählte physikalische Meßgröße der Summe der vorgegebenen Alkane kann je nach Anwendungsfall beliebig gewählt werden. Z.B. kann statt mit dem molaren Brennwert mit der molaren Masse, der Dichte oder der Schallgeschwindigkeit gearbeitet werden.

Vorteilhafterweise können als Funktionen zur Bestimmung der Anteile der einzelnen Alkane, ausgenommen Methan, Polynone, vorzugsweise 2. Ordnung, verwendet werden.

Bei einem bevorzugten Ausführungsbeispiel wird der Methan-, Ethan-, Propan-, Isobutan-, n-Butan-, Isopentan-, n-Pentan-, Hexan-, Heptan- und Oktananteil mit Hilfe der Funktionen bestimmt. Es hat sich gezeigt, daß der Anteil aller weiteren Alkane, insbesondere bei natürlichem Brenngas, vernachlässigt werden kann. Der Zusammenhang zu dem molaren Brennwert der Summe der vorgegebenen Alkane lautet in diesem Fall z.B.:

$$X_{C_2H_6} = [\alpha_1 (H_{CH} - H_{CH_4}) + \beta_1 (H_{CH} - H_{CH_4})^2] x_{CH} \quad (1.1)$$

$$X_{C_3H_8} = [\alpha_2 (H_{CH} - H_{CH_4}) + \beta_2 (H_{CH} - H_{CH_4})^2] x_{CH} \quad (1.2)$$

$$X_{i-C_4H_{10}} = [\alpha_3 (H_{CH} - H_{CH_4}) + \beta_3 (H_{CH} - H_{CH_4})^2] x_{CH} \quad (1.3)$$

$$X_{n-C_4H_{10}} = [\alpha_4 (H_{CH} - H_{CH_4}) + \beta_4 (H_{CH} - H_{CH_4})^2] x_{CH} \quad (1.4)$$

$$X_{i-C_5H_{12}} = [\alpha_5 (H_{CH} - H_{CH_4}) + \beta_5 (H_{CH} - H_{CH_4})^2] x_{CH} \quad (1.5)$$

$$X_{n-C_5H_{12}} = [\alpha_6 (H_{CH} - H_{CH_4}) + \beta_6 (H_{CH} - H_{CH4})^2] x_{CH} \quad (1.6)$$

$$X_{n-C_6H_{14}} = [\alpha_7 (H_{CH} - H_{CH_4}) + \beta_7 (H_{CH} - H_{CH_4})^2] x_{CH} \quad (1.7)$$

$$X_{n-C_7H_{16}} = [\alpha_8 (H_{CH} - H_{CH_4}) + \beta_8 (H_{CH} - H_{CH_4})^2] x_{CH} \quad (1.8)$$

$$X_{n-C_8H_{18}} = [\alpha_9 (H_{CH} - H_{CH_4}) + \beta_9 (H_{CH} - H_{CH_4})^2] x_{CH} \quad (1.9)$$

Hierbei sind $\alpha_i$ und $\beta_i$ Konstanten und

$$H_{CH_4}$$

der molare Brennwert von Methan. Die Variable $H_{CH}$ steht für den molaren Brennwert der Summe der vorgegebenen Alkane ($H_{CH} = \Sigma X_{CH,i} H_{CH,i}$). Der Methananteil wird in diesem Fall wie folgt bestimmt:

$$X_{CH_4} = X_{CH} - (X_{C_2H_6} + X_{C_3H_8} + X_{i\text{-}C_4H_{10}} + X_{n\text{-}C_4H_{10}} + X_{i\text{-}C_5H_{12}} + X_{n\text{-}C_5H_{12}} + X_{n\text{-}C_6H_{14}} + X_{n\text{-}C_7H_{16}} + X_{n\text{-}C_8H_{18}}) \tag{2}$$

Eine Weiterbildung der Erfindung ist dadurch gekennzeichnet, daß den Verfahrensschritten a) und b) mehrere Meßzyklen vorgeschaltet werden, bei denen der Schritt a) mit mehreren Referenzgasen durchgeführt wird, deren Zusammensetzung und deren ausgewählte physikalische Meßgröße der Summe der vorgegebenen Alkane bekannt ist, daß aus den bei den Referenzgasen erfaßten Meßsignalen die Konstanten, z.B. Koeffizienten, der den Anteil der Alkane, ausgenommen Methan, beschreibenden Funktionen bestimmt werden, daß die Konstanten der Funktionen in Zuordnung zu den jeweiligen Alkanen gespeichert werden und daß der Anteil der Alkane, ausgenommen Methan, aus einem späteren Meßzyklus an Brenngas unbekannter Zusammensetzung mit Hilfe der Funktionen bestimmt wird.

Auf diese Weise können die Konstanten $\alpha_i$ und $\beta_i$ mit Hilfe bereits von zwei Referenzzyklen für alle natürlichen Erdgase aufgefunden werden. Zur Erhöhung der Meßgenauigkeit, können beliebig viele Referenzzyklen durchgeführt werden. Der Aufwand bleibt auch bei einer großen Anzahl von Referenzzyklen verhältnismäßig gering, da die Konstanten $\alpha_i$ und $\beta_i$ nur einmal bestimmt werden müssen.

Vorteilhafterweise wird der molare Brennwert der Summe der vorgegebenen Alkane als lineare Funktion der molaren Masse der Summe der vorgegebenen Alkane bestimmt. Die diesbezügliche Gleichung lautet für die natürlichen Erdgase:

$$M_{CH} = b_0 + b_1 \ H_{CH} \tag{3}$$

Hierbei ist $M_{CH}$ die molare Masse der Summe der vorgegebenen Alkane. $b_0$ und $b_1$ sind Konstanten und betragen -2.68454 bzw. 0.0210273.

Der Stickstoffanteil sowie die molare Masse der Summe der vorgegebenen Alkane können aus der molaren Masse des Brenngases unter der Annahme bestimmt werden, daß das Brenngas nur aus einer vorgegebenen Anzahl von Alkanen, aus Stickstoff und aus Kohlenstoffdioxid besteht. Die zugehörigen Gleichungen haben folglich die Form:

$$X_{N_2} = 1 - X_{CH} - X_{CO_2} \tag{4}$$

Hierbei geben

$$X_{N_2} \ \text{und} \ X_{CO_2}$$

den Anteil von Stickstoff bzw. Kohlenstoffdioxid an. Daraus folgt für die molare Masse des Brenngases ($M_{mix}$):

$$M_{mix} = X_{CH}M_{CH} + X_{N2}M_{N_2} + X_{CO_2}M_{CO_2} \tag{5}$$

Zur Erhöhung der Meßgenauigkeit kann alternativ angenommen werden, daß das Brenngas außerdem Wasserstoff und/oder Kohlenstoffmonoxid enthält. Werden sowohl der Wasserstoffals auch der Kohlenstoffmonoxidanteil berücksichtigt, so lauten die Gleichungen (4) und (5):

$$X_{N_2} = 1 - X_{CH} - X_{CO_2} - X_{H_2} - X_{CO} \tag{4'}$$

$$M_{mix} = X_{CH}M_{CH} + X_{N2}M_{N_2} + X_{CO_2}M_{CO_2} + X_{H_2}M_{H_2} + X_{CO}M_{CO} \tag{5'}$$

Vorzugsweise wird der molare Brennwert der Summe der vorgegebenen Alkane aus der erfaßten Dichte, dem erfaßten Brennwert, der molaren Masse des Brenngases, dem Anteil der Summe der vorgegebenen Alkane und ggf. aus dem Anteil sowie dem molaren Brennwert von Wasserstoff und Kohlenstoffmonoxid abgeleitet. Die zugehörige Gleichung lautet:

$$H_S = \rho_n (X_{CH}H_{CH} + X_{H2}H_{H2} + X_{CO}H_{CO}) M_{mix}^{-1} \tag{6}$$

Hierbei ist $H_S$ der erfaßte Brennwert und $\rho_n$ die erfaßte Dichte bei Normbedingungen.

Ein bevorzugtes Ausführungsbeispiel ist dadurch gekennzeichnet, daß der Realgasfaktor aus der Gaszusammensetzung abgeleitet wird und daß die molare Masse des Brenngases aus der Dichte und dem Realgasfaktor abgeleitet wird.

Der Realgasfaktor ($Z_n$) kann beispielsweise mit der im Stand der Technik bekannten AGA8-DC92-Zustandsgleichung, mit der DIN 51857 oder der ISO 6976 bestimmt werden:

$$Z_n = f(T_n, p_n, x_i) \tag{7}$$

Die Dichte unter Normbedingungen kann daraus wie folgt bestimmt werden:

$$\rho_n = \frac{p_n M_{mix}}{Z_n R_m T_n} \tag{8}$$

Eine Weiterbildung der Erfindung ist dadurch gekennzeichnet, daß aus der Zusammensetzung des Brenngases ein Wert für dessen Dichte abgeleitet wird, daß die Differenz zwischen dem abgeleiteten und dem erfaßten Wert der Dichte gebildet wird, daß falls die Differenz einen vorgegebenen Schwellwert überschreitet, der Anteil wenigstens einer der zu bestimmenden Komponenten des Brenngases abgeändert festgesetzt wird, daß auf der Basis des abgeänderten Wertes erneut die Zusammensetzung, die Dichte und die Differenz bestimmt werden und daß die letzten beiden Schritte solange wiederholt werden, bis die Differenz unterhalb des Schwellwertes liegt.

Es hat sich gezeigt, daß auf diese Weise die Gaszusammensetzung besonders schnell bestimmt werden kann. Sollen alle oben angegebenen 16 Gleichungen (1.1 bis 8) berücksichtigt werden, so enthalten diese bis zu 16 Unbekannte, nämlich

$$X_{CH_4}, \ X_{N_2}, \ X_{C_2H_6}, \ X_{C_3H_8}, \ \cdots \ X_{n-C_8H_{18}},$$

$M_{mix}$, $X_{CH}$, $H_{CH}$, $M_{CH}$, $Z_n$. Zur Lösung dieser 16 Gleichungen kann ein Wert, z.B. die Dichte, abgeleitet und mit dem zugehörigen erfaßten Wert verglichen werden.

Das erfindungsgemäße Verfahren kann zur Bestimmung des Realgasfaktors, der Dichte, der Schallgeschwindigkeit, der Enthalpie, der Methanzahl oder des Wobbe-Indizes des Brenngases verwendet werden. Die mit dem erfindungsgemäßen Verfahren bestimmten physikalischen Größen haben im wesentlichen die gleiche Güte wie die mit Hilfe einer Gaschromatographie bestimmten Werte. Erfindungsgemäß gelingt es mit Hilfe von lediglich zwei Meßgrößen, z.B. Brennwert und Dichte unter Normbedingungen, eine Vielzahl von verfahrenstechnischen Berechnungen durchzuführen. Zunächst können Zustandsänderungen in Gasspeichern bzw. Speichervolumen festgestellt werden. Außerdem können die relevanten Gastransportdaten, wie z.B. Temperatur oder Druckabfall ermittelt werden. Für erdgasbetriebene Fahrzeuge kann die erforderliche Auslegung der Gasbefüllstationen berechnet werden. Füllstandsmessungen können mit dem erfindungsgemäßen Verfahren kontrolliert und ausgelegt werden.

Auch im Zusammenhang mit Wärmetauschern ist die Erfindung von großem Vorteil. Die Auslegung von Wärmetauschern kann mit dem erfindungsgemäßen Verfahren berechnet werden. Leistungsmessungen an Wärmetauschern können mit dem Verfahren ausgewertet werden. Schließlich können Verdichterkennfelder und Verdichterleistungen mit dem erfindungsgemäßen Verfahren bestimmt werden.

Bei sämtlichen vorgenannten Anwendungen sind bisher kostspielige Gaschromatographie-Untersuchungen erforderlich.

Auch die Methanzahl kann mit dem erfindungsgemäßen Verfahren bestimmt werden. Werden als Eingangsmeßsignale für das erfindungsgemäße Verfahren diejenigen Gasbeschaffenheitsdaten verwendet, die ursprünglich für Abrechnungszwecke gemessen wurden, so kann die Methanzahl im wesentlichen mit der gleichen Genauigkeit wie bei Einsatz eines Gaschromatographen bestimmt werden. Die Abweichung der Methanzahlen beträgt weniger als 2%.

Alternativ können die Gasbeschaffenheitsdaten wie z.B. Brennwert und Dichte im Normzustand aus einer Netzsimulation bestimmt werden. Netzsimulationen können derzeit den Brennwert mit einer Unsicherheit von weniger als 1 Prozent und die Dichte im Normzustand mit einer Unsicherheit von 1,5 Prozent genau bestimmen. Der Kohlenstoffdioxidanteil kann in diesem Fall einfach als mittlerer Wert von 1 mol % festgesetzt werden. Die auf diese Weise nach dem erfindungsgemäßen Verfahren bestimmte Methanzahl stimmt mit der bei Verwendung eines Gaschromatographen ermittelten Methanzahl im allgemeinen noch besser als zwei Prozent überein. Diese Genauigkeit genügt für die meisten Anwendungsbereiche.

Bei Anwendungen, bei denen ein Volumen- oder Massenstromzähler vorhanden ist, ist es vorteilhaft, das Meßsignal für die Schallgeschwindigkeit bei diesem Volumen- oder Massenstromzähler abzugreifen.

Bei Verwendung der für Abrechnungszwecke gemessenen Gasbeschaffenheitsdaten oder der Netzsimulationen gelingt es, ohne zusätzliche Messungen Brenngasabnehmer jederzeit über aktuelle und ggf. zukünftige Schwankungen der Methan-Zahl zu informieren. Das Gastransportnetz kann außerdem ohne Mehraufwand flexibler gesteuert werden.

Weitere vorteilhafte Ausführungsformen der Erfindung sind in den Unteransprüchen gekennzeichnet.

Im folgenden wird die Erfindung anhand eines bevorzugten Ausführungsbeispiels mit Hilfe der beigefügten Zeichnung näher erläutert.

In der Zeichnung zeigt:

Fig. 1    ein Meßdiagramm, in dem der Stoffmengenanteil von Ethan und Propan für verschiedene Gase gegen den molaren Brennwert der Summe der Kohlenwasserstoffe aufgetragen ist, und

Fig. 2    ein Ablaufdiagramm zur Bestimmung der Gaszusammensetzung gemäß einem Ausführungsbeispiel des erfindungsgemäßen Verfahrens.

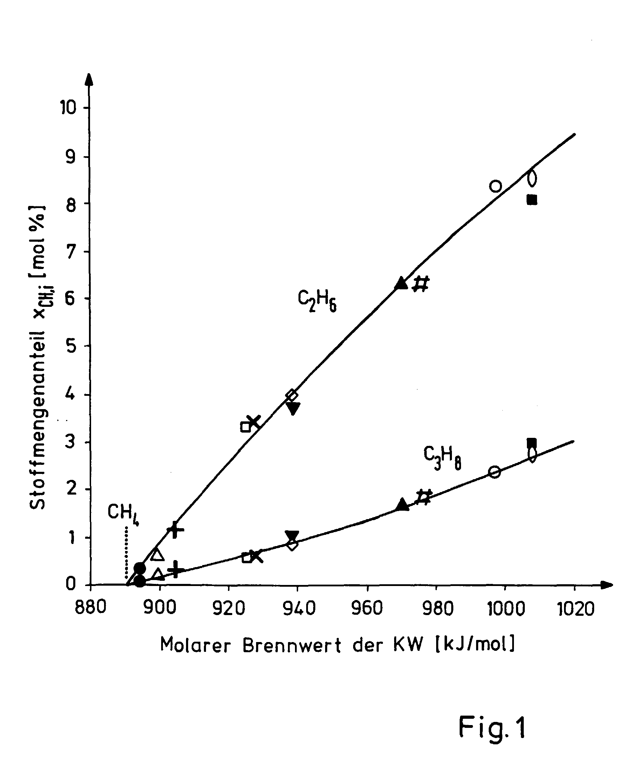

In Fig. 1 ist der molare Brennwert der Summe der Alkane ($H_{CH}$) auf der x-Achse und der Stoffmengenanteil von Ethan ($C_2H_6$) und Propan ($C_3H_8$) auf der y-Achse aufgetragen. Für verschiedene natürliche Erdgase wurden die entsprechenden Meßwerte ermittelt und aufgetragen. Der Anteil von sowohl Ethan als auch Propan wurde durch ein Polynom 2. Ordnung angenähert. Wie die Fig. 1 zeigt, kann sowohl der Anteil von Ethan als auch von Propan überraschend gut durch ein vom molaren Brennwert der Summe der Alkane abhängiges Polynom 2. Ordnung angenähert werden. Entsprechendes gilt für die weiteren Alkane bis zum Octan. Die Meßwerte für diese Alkane sind aus Gründen der Übersichtlichkeit in Fig. 1 nicht dargestellt.

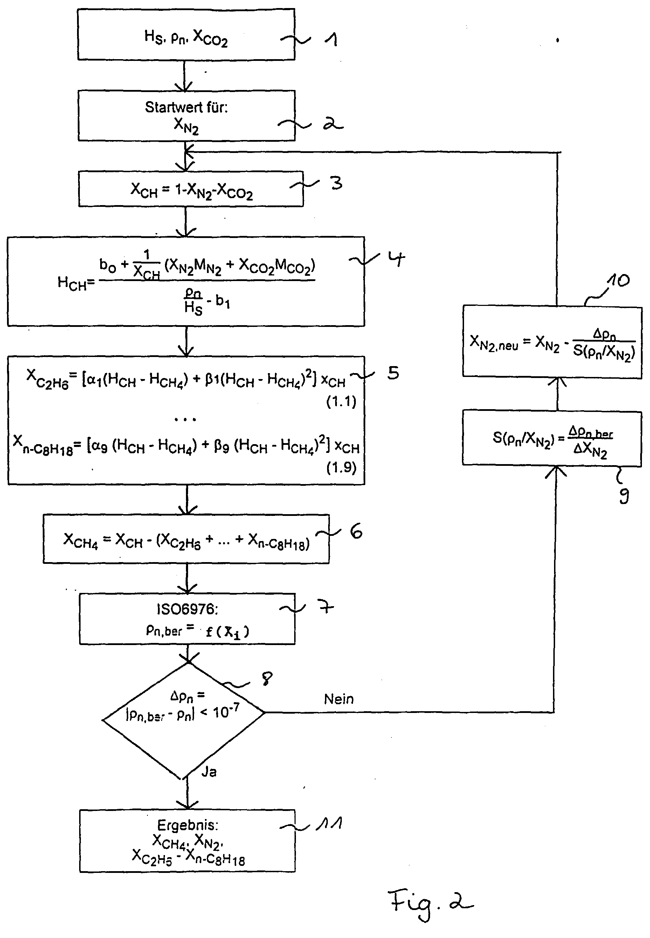

Fig. 2 zeigt ein Ablaufdiagramm zur Bestimmung der Gaszusammensetzung gemäß einem bevorzugten Ausführungsbeispiel. Im Schritt 1 werden der Brennwert des Brenngases $H_S$, die Dichte unter Normbedingungen $\rho_n$ sowie der Kohlenstoffdioxidanteil

$$x_{CO_2}$$

gemessen. Im Schritt 2 wird ein Startwert für den Stickstoffanteil

$$x_{N_2}$$

festgelegt. Aus dem erfaßten Kohlenstoffdioxidanteil und dem Startwert für den Stickstoffanteil wird dann im Schritt 3 der Anteil der Summe der Alkane $H_{CH}$ bestimmt. Mit Hilfe des berechneten Wertes des Anteils der Summe der Alkane $x_{CH}$ kann dann im Schritt 4 der molare Brennwert der Summe der Alkane $H_{CH}$ bestimmt werden. Die dazu verwendete Gleichung kann aus den Gleichungen (2) bis (6) abgeleitet werden. Hierbei muß berücksichtigt werden, daß bei diesem Ausführungsbeispiel der Kohlenstoffmonoxid und Wasserstoffanteil vernachlässigt werden und folglich gleich Null zu setzen sind.

Im Schritt 5 wird dann mit Hilfe des bestimmten molaren Brennwerts der Summe der Alkane $H_{CH}$ der Anteil der Alkane, ausgenommen Methan, mit Hilfe der Gleichungen (1.1) bis (1.9) bestimmt. Im Schritt 6 wird der Methananteil

$$x_{CH_4}$$

gemäß Gleichung (2) bestimmt. Im Schritt 7 wird aus der berechneten Zusammensetzung des Gases die Dichte bei Normbedingungen $\rho_{n,ber}$ berechnet. Dies geschieht mit Hilfe der bekannten ISO 6976, und zwar über den Realgasfaktor gemäß den Gleichungen (8) und (9). Im Schritt 8 wird festgestellt, ob der Betrag der Differenz zwischen berechneter Normdichte

$\rho_{n,ber}$ und im Schritt 1 erfaßter Normdichte kleiner als der auf $10^{-7}$ festgelegte Schwellwert ist. Falls nein, wird das Verfahren mit dem Schritt 9 fortgesetzt.

Im Schritt 9 wird die Empfindlichkeit bzw. Sensitivity

$$S(\rho_n/x_{N_2})$$

bestimmt. Dazu wird ein

$$\Delta x_{N_2}$$

festgesetzt, z.B. 0,01%, und für einen entsprechend variierten Wert des Stickstoffanteils mit der Hilfe der Schritte 3-8 ein zweiter Wert für $P_{n,ber}$ bestimmt. Die Differenz dieser beiden berechneten Werte der Dichte, $\Delta\rho_{n,ber}$, wird dann geteilt durch

$$\Delta x_{N_2} \, .$$

Im Schritt 10 wird dann ein neuer Wert für den Stickstoffanteil

$$x_{N_2} \, ,$$

neu festgesetzt, und zwar indem von dem Startwert des Stickstoffanteils der Quotient aus dem im Schritt 8 ermittelten $\Delta\rho_n$ und

$$S(\rho_n / x_{N_2})$$

abgezogen wird. Mit dem neu festgesetzten Wert für den Stickstoffanteil werden die Schritte 3 bis 8 dann wiederholt. Falls im Schritt 8 erneut der Grenzwert von $\Delta\rho_n$ überschritten wird, werden erneut die Schritte 9, 10 und 3 bis 8 durchgeführt. Erst wenn im Schritt 8 der Grenzwert von $10^{-7}$ von $\Delta\rho_n$ eingehalten wird, stehen die Anteile der Alkane sowie der Stickstoffanteil mit der gewünschten Genauigkeit fest.

Der Startwert für den Stickstoffanteil kann im Schnitt 2 z.B. dadurch bestimmt werden, daß man als molaren Brennwert der Summe der Alkane den molaren Brennwert von Methan verwendet

$$(H_{CH} = H_{CH_4}) \, .$$

Setzt man diesen Brennwert von Methan in die im Schritt 4 angegebene Gleichung ein, so erhält man nach geeigneter Auflösung einen Startwert für den Stickstoffanteil.

Im Rahmen des Erfindungsgedankens sind zahlreiche Variationsmöglichkeiten gegeben. Die zur Bestimmung der Gaszusammensetzung benötigten Meßgrößen können entweder gemessen werden oder vorzugsweise auf Meßwerten beruhenden Meßsimulationen entnommen werden. Statt der absoluten Dichte kann auch die auf trockene Luft bezogene relative Dichte erfaßt werden. Die zu bestimmenden, vorgegebenen Alkane können jeweils für einen speziellen Anwendungsfall beliebig gewählt werden. Schließlich können die angegebenen 16 Gleichungen auf jede beliebige Weise und in beliebiger Reihenfolge aufgelöst werden.

**Patentansprüche**

1.    Verfahren zur Bestimmung der Gaszusammensetzung von Brenngas, insbesondere von Erdgas, wobei

a) der Brennwert oder die Dielektrizitätskonstante des Brenngases unter Referenz- oder Betriebsbedingungen und die Dichte oder die Schallgeschwindigkeit des Brenngases unter Referenz- oder Betriebsbedingungen erfaßt werden und
b) aus den erfaßten Zwei Meßsignalen die Gaszusammensetzung abgeleitet wird.

2.    Verfahren nach Anspruch 1, dadurch gekennzeichnet, daß als Referenzbedingungen für die Messung des Brennwertes oder der Dielektrizitätskonstanten und/oder der Dichte und/oder der Schallgeschwindigkeit Normbedingungen eingestellt werden.

3.    Verfahren nach Anspruch 1 oder 2, dadurch gekennzeichnet, daß im Schritt a) zusätzlich wenigstens eine der Meßgrößen Druck, Temperatur, Kohlenstoffdioxidanteil, Kohlenstoffmonoxid- und Wasserstoffanteil des Brenngases erfaßt wird.

4. Verfahren nach einem der Ansprüche 1 bis 3, dadurch gekennzeichnet, daß den Verfahrensschritten a) und b) mehrere Meßzyklen vorgeschaltet werden, bei denen der Schritt a) mit mehreren Referenzgasen bekannter Zusammensetzung durchgeführt wird; daß aus dem Verhältnis der bei den Referenzgasen erfaßten Meßsignale eine der Zahl der Meßzyklen entsprechende Zahl von Referenzsignalmustern in Zuordnung zu den bekannten Zusammensetzungen gespeichert wird; und daß das Signalmuster aus einem späteren Meßzyklus an Brenngas unbekannter Zusammensetzung mit den Referenzsignalmustern zur Zuordnung einer bestimmten Zusammensetzung verglichen wird.

5. Verfahren nach einem der Ansprüche 1 bis 4, dadurch gekennzeichnet,

daß der jeweilige Anteil einer vorgegebenen Anzahl von Alkanen, einschließlich Methan, dadurch bestimmt wird,
daß der Anteil der einzelnen Alkane, ausgenommen Methan, mit Hilfe jeweils einer zugehörigen, von einer ausgewählten physikalischen Meßgröße, vorzugsweise dem molaren Brennwert, der Summe der vorgegebenen Alkane abhängigen Funktion bestimmt wird und
daß der Methananteil aus der Differenz zwischen dem Anteil der Summe der vorgegebenen Alkane und der Summe der durch die Funktionen bestimmten Anteile der Alkane bestimmt wird.

6. Verfahren nach Anspruch 5, dadurch gekennzeichnet, daß als Funktion ein Polynon, vorzugsweise 2. Ordnung, verwendet wird.

7. Verfahren nach Anspruch 5 oder 6, dadurch gekennzeichnet, daß der Methan-, Ethan-, Propan-, Isobutan-, n-Butan-, Isopentan-, n-Pentan-, Hexan-, Heptan- und Oktananteil mit Hilfe der Funktionen bestimmt wird.

8. Verfahren nach einem der Ansprüche 5 bis 7, dadurch gekennzeichnet, daß den Verfahrensschritten a) und b) mehrere Meßzyklen vorgeschaltet werden, bei denen der Schritt a) mit mehreren Referenzgasen durchgeführt wird, deren Zusammensetzung und deren ausgewählte physikalische Meßgröße der Summe der vorgegebenen Alkane bekannt ist,

daß aus den bei den Referenzgasen erfaßten Meßsignalen die Konstanten, z.B. Koeffizienten, der den Anteil der Alkane, ausgenommen Methan, beschreibenden Funktionen bestimmt werden,
daß die Konstanten der Funktionen in Zuordnung zu den jeweiligen Alkanen gespeichert werden und
daß der Anteil der Alkane, ausgenommen Methan, aus einem späteren Meßzyklus an Brenngas unbekannter Zusammensetzung mit Hilfe der Funktionen bestimmt wird.

9. Verfahren nach einem der Ansprüche 1 bis 8, dadurch gekennzeichnet, daß die ausgewählte physikalische Meßgröße, vorzugsweise der molare Brennwert, der Summe der vorgegebenen Alkane als lineare Funktion einer anderen physikalischen Meßgröße, vorzugsweise der molaren Masse der Summe der vorgegebenen Alkane bestimmt wird.

10. Verfahren nach einem der Ansprüche 1 bis 9, dadurch gekennzeichnet, daß unter der Annahme, daß das Brenngas nur aus einer vorgegebenen Anzahl von Alkanen, aus Stickstoff, und Kohlenstoffdioxid besteht, sowohl der Stickstoffanteil als auch mit Hilfe der molaren Masse des Brenngases die molare Masse der Summe der vorgegebenen Alkane bestimmt werden.

11. Verfahren nach einem der Ansprüche 1 bis 9, dadurch gekennzeichnet, daß unter der Annahme, daß das Brenngas nur aus einer vorgegebenen Anzahl von Alkanen, aus Stickstoff, Kohlenstoffdioxid sowie Wasserstoff und/oder Kohlenstoffmonoxid besteht, sowohl der Stickstoffanteil als auch mit Hilfe der molaren Masse des Brenngases die Molmasse der Summe der vorgegebenen Alkane bestimmt werden.

12. Verfahren nach einem der Ansprüche 1 bis 11, dadurch gekennzeichnet, daß der molare Brennwert der Summe der vorgegebenen Alkane aus der erfaßten Dichte, dem erfaßten Brennwert, der molaren Masse des Brenngases, dem Anteil der Summe der vorgegebenen Alkane und ggf. aus dem Anteil sowie dem molaren Brennwert von Wasserstoff und Kohlenstoffmonoxid abgeleitet wird.

13. Verfahren nach einem der Ansprüche 1 bis 12, dadurch gekennzeichnet, daß der Realgasfaktor aus der Zusammensetzung abgeleitet wird und

daß die molare Masse des Brenngases aus der Dichte und dem Realgasfaktor abgeleitet wird.

14. Verfahren nach einem der Ansprüche 1 bis 13, dadurch gekennzeichnet,

daß aus der Zusammensetzung des Brenngases ein Wert für dessen Dichte abgeleitet wird,
daß die Differenz zwischen dem abgeleiteten und dem erfaßten Wert der Dichte gebildet wird,
daß falls die Differenz einen vorgesehenen Schwellwert überschreitet, der Anteil wenigstens einer der zu bestimmenden Komponenten des Brenngases abgeändert festgesetzt wird,
daß auf der Basis des abgeänderten Wertes erneut die Zusammensetzung, die Dichte und die Differenz bestimmt werden und
daß die letzten beiden Schritte solange wiederholt werden, bis die Differenz unterhalb des Schwellwertes liegt.

15. Verfahren nach einem der Ansprüche 1 bis 14, dadurch gekennzeichnet, daß die Dielektrizitätskonstante und die Dichte oder die Schallgeschwindigkeit unter Referenzbedingungen in einer gemeinsamen Meßumgebung erfaßt werden.

16. Verfahren nach einem der Ansprüche 1 bis 15, dadurch gekennzeichnet, daß als Referenzbedingung für die Messung der Dielektrizitätskonstanten ein Referenzdruck von wenigstens 1 MPa eingestellt wird.

17. Verfahren nach einem der Ansprüche 1 bis 16, dadurch gekennzeichnet, daß das Meßsignal für die Schallgeschwindigkeit von einem Volumen- oder Massenstromzähler abgegriffen wird.

18. Verwendung des Verfahrens nach einem der Ansprüche 1 bis 17 zur Bestimmung des Realgasfaktors, der Dichte, der Schallgeschwindigkeit, der Enthalpie, der Methanzahl oder des Wobbe-Indizes des Brenngases.

Fig.1

$$H_S, \rho_n, X_{CO_2}$$   1

$$\text{Startwert für:}\\ X_{N_2}$$   2

$$X_{CH} = 1 - X_{N_2} - X_{CO_2}$$   3

$$H_{CH} = \dfrac{b_0 + \dfrac{1}{X_{CH}}(X_{N_2}M_{N_2} + X_{CO_2}M_{CO_2})}{\dfrac{\rho_n}{H_S} - b_1}$$   4

$$X_{C_2H_6} = [\alpha_1(H_{CH} - H_{CH_4}) + \beta_1(H_{CH} - H_{CH_4})^2]\, x_{CH}$$ (1.1)

$$\ldots$$

$$X_{n\text{-}C_8H_{18}} = [\alpha_9(H_{CH} - H_{CH_4}) + \beta_9(H_{CH} - H_{CH_4})^2]\, x_{CH}$$ (1.9)   5

$$X_{CH_4} = X_{CH} - (X_{C_2H_6} + \ldots + X_{n\text{-}C_8H_{18}})$$   6

$$\text{ISO6976:}\\ \rho_{n,ber} = f(X_i)$$   7

$$\Delta\rho_n = |\rho_{n,ber} - \rho_n| < 10^{-7}$$   8

Nein

Ja

$$X_{N2,neu} = X_{N_2} - \dfrac{\Delta\rho_n}{S(\rho_n/X_{N_2})}$$   10

$$S(\rho_n/X_{N_2}) = \dfrac{\Delta\rho_{n,ber}}{\Delta X_{N_2}}$$   9

$$\text{Ergebnis:}\\ X_{CH_4}, X_{N_2},\\ X_{C_2H_6} - X_{n\text{-}C_8H_{18}}$$   11

Fig. 2